# EUROPEAN PATENT SPECIFICATION

(11) **EP 3 410 964 B1**
(45) Date of publication and mention of the grant of the patent: **18.03.2020**
(21) Application number: 16704052.6
(22) Date of filing: 03.02.2016
(51) Int. Cl.: A61B 17/64, A61B 17/66

(54) **EXTERNAL ORTHOPEDIC DEVICE**
EXTERNE ORTHOPÄDISCHE VORRICHTUNG
DISPOSITIF ORTHOPÉDIQUE EXTERNE

(43) Date of publication of application: 12.12.2018
(73) Proprietor: Citieffe S.r.L., 40012 Calderara Di Reno (IT)
(72) Inventor: DOVESI, Alan, 40138 Bologna (IT); SCOCCIANTI, Alberto, 40036 Rioveggio - Monzuno (Bologna) (IT); SATIZABAL, Carlos, Barrio Nicolás de Federman, Bogota' (CO)
(74) Representative: Milli, Simone
(86) International application number: PCT/IB2016/050541
(87) International publication number: WO 2017/134486

(56) References cited:
- US-A- 5 601 551
- US-A1- 2010 222 778

## Description

### Technical field

This invention relates to an orthopedic device.
In particular, the invention relates to an external orthopedic device for the treatment of the long bones of the limbs.
Certain diseases require treatment of the long bones of the limbs by means of osteotomy, with the formation of two or more bone stumps or segments and subsequent relative movement of these stumps or segments.
These treatments include lengthening of a limb and the correction of anatomical deformities.
The lengthening of a limb may be necessary for a number of reasons, not just, for example, for esthetic reasons related to an increase in height.
Unlike what one might think, in fact, most bone lengthening treatments are due to diseases that necessitated the removal of portions, sometimes quite large portions, of bone.

### Background art

In order to functionally manage the long port-operative period, devices are available that are equipped with multiple clamps that hold bone screws, with each clamp stably connected to a respective bone stump and sliding along a guide mechanism.
The relative repositioning of the various bone stumps or segments is actively managed by means of these devices, preferably by the patient him/herself.
In the simplest cases, the surgical operation consists of osteotomy and subsequent lengthening of the two bone stumps.

When the lengthening must be considerable, it is preferable, above all to speed up the process, to perform two osteotomies, thereby obtaining three stumps and dividing the lengthening between these two osteotomies.
The underlying disease sometimes requires the movement of a bone stump from one part (for example proximal) to another (for example distal). One of the advantages connected with the use of these external devices compared with internal ones is that they are easy to remove in the case of infections, and easy to progressively adjust and correct.
When the surgeon believes there may be a risk of infection, he will therefore opt for an external device.
External devices are normally used for long bones: femur, tibia and humerus, varying only the length of the guide mechanism.
It is then the patient who periodically carries out the necessary operations on the device due to the distraction/coarctation of the bone stumps.
Treatments of this type take a considerable amount of time, sometimes several months, and the patient is therefore obliged to use the device for a long time.
Document US 2010/0222778 discloses an external orthopaedic device for limb bone treatment of known type.
Prior art external orthopedic devices are not without drawbacks.
The first of these drawbacks is due to the complexity of the operations needed to move the clamps connected to the various bone stumps. This complexity creates stress for the patient and involves a high risk of error.
Additional drawbacks are connected with the constructional form of the guide mechanism, too often designed for high mechanical performance but not taking into account, for example, the requirements of hygiene and cleanliness, indispensable in a medical device.
One example of such drawbacks is the configuration of the guide mechanism, which in prior art devices often includes dovetail sections, a configuration that constitutes a receptacle for dirt.

### Disclosure of the invention

The aim of this invention is to provide an external orthopedic device that overcomes the prior art drawbacks described above.
More specifically, the aim of this invention is to provide an external orthopedic device which is practical to use and limits the accumulation of dirt.
The aims indicated above are substantially achieved by an external orthopedic device comprising the technical features disclosed in one or more of the accompanying claims.

### Brief description of drawings

The technical features of the invention, according to the aforesaid aims, are clearly disclosed in the claims below, and their advantages will become more evident in the detailed description that follows, with reference to the accompanying drawings which represent one embodiment provided as a non-binding example, wherein:
Figures 1a and 1b are schematic prospective views from above of a first form of the orthopedic device according to the invention in two different configurations;
Figures 2a to 2c are schematic, prospective cross-section views of a detail of the device shown in figures 1a and 1b;
Figures 3a to 3f are, respectively, schematic, prospective, cross-section elevational views of a construction variation of the detail shown in Figures 2a to 2c;
Figures 4a to 4g are, respectively, schematic, prospective, cross-section elevational views of another construction variation of the detail shown in Figures 2a to 2c;
Figures 5a to 5f are, respectively, schematic, prospective, cross-section, plan and elevational views of another construction variation of the detail shown in Figures 2a to 2c;

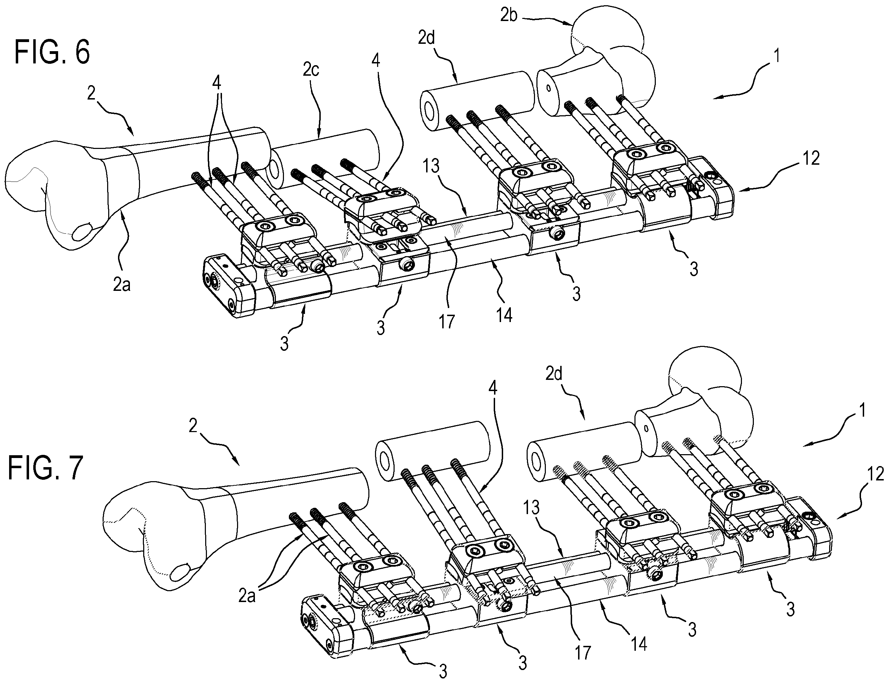

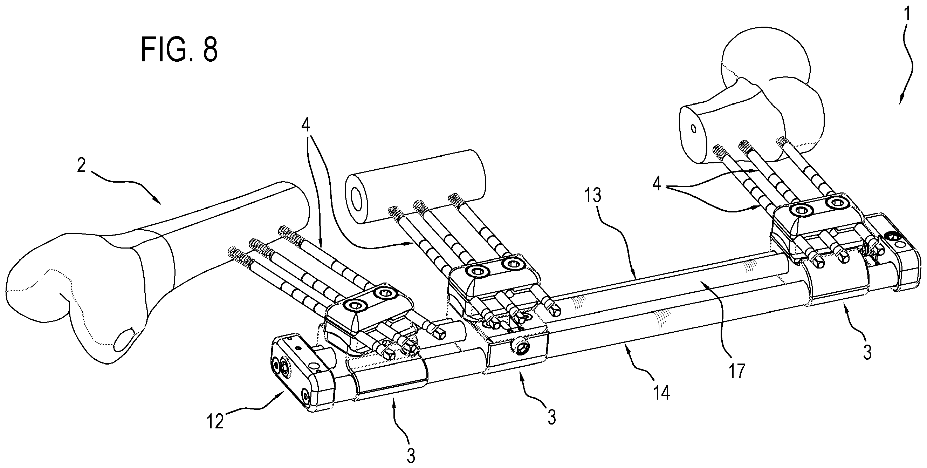

Figures 6 to 8 show variant embodiments and configurations of the device shown in Figures 1a and 1b in respective schematic perspective views from above.

### Detailed description of preferred embodiments of the invention

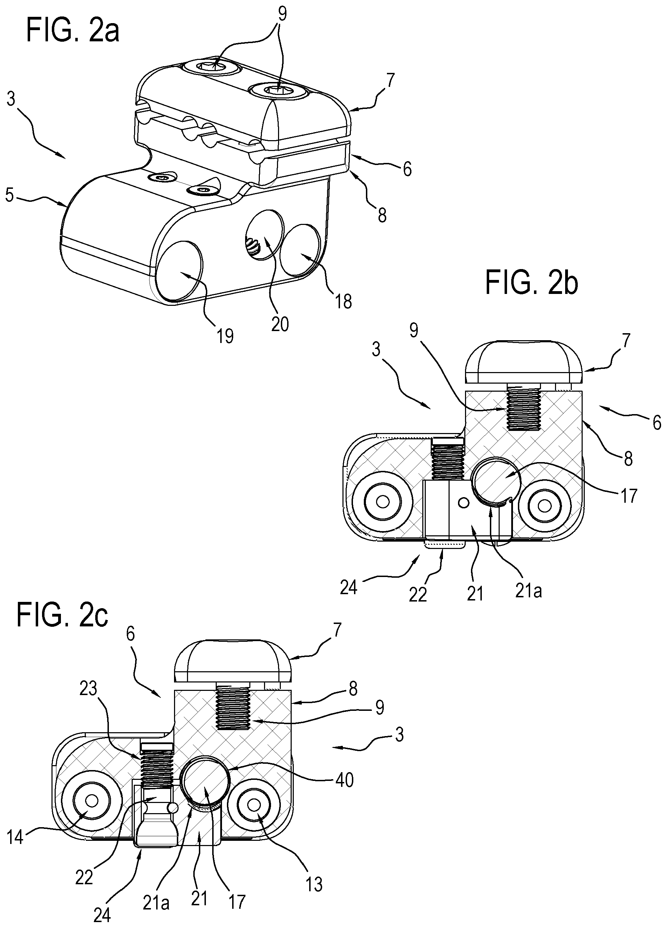

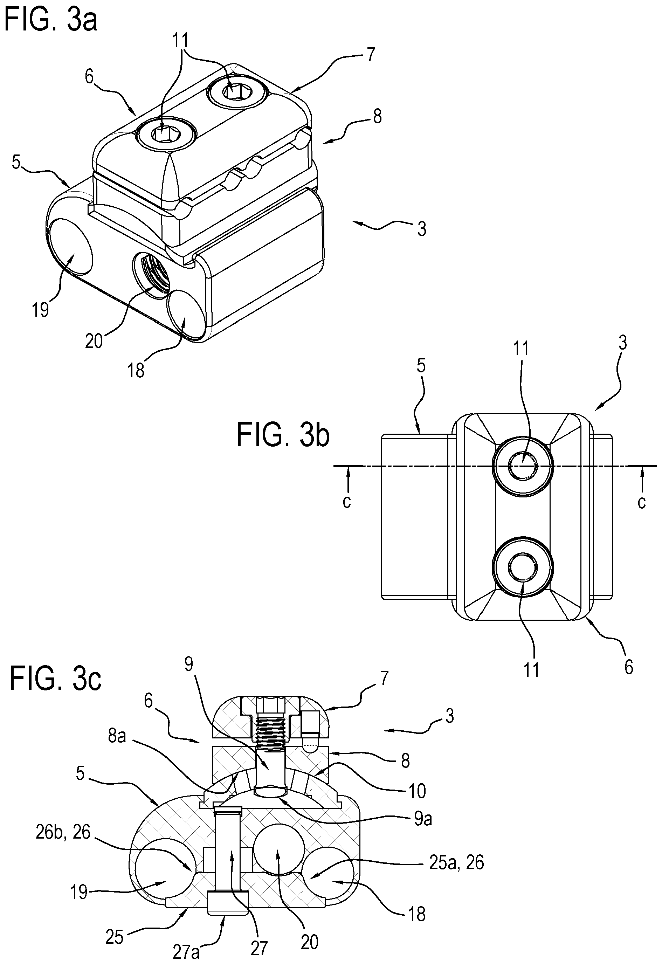

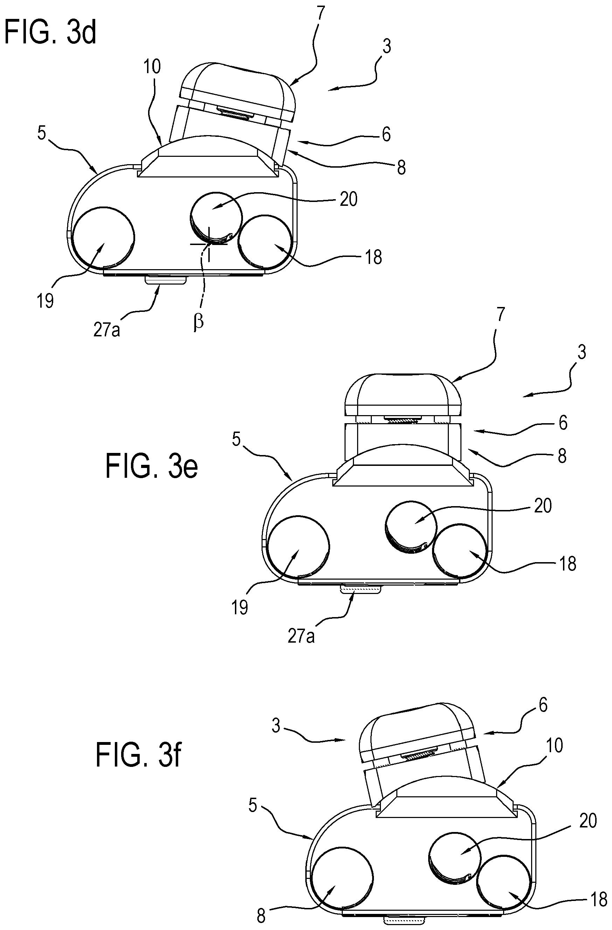

With reference to the accompanying drawings, the numeral 1 denotes in its entirety an external orthopedic device for the treatment of limb bones. By way of example, the accompanying drawings show a femur 2 cut into a plurality of bone segments indicated by the numerals 2a to 2d.
The device 1 comprises a plurality of clamps 3 holding bone screws 4 to be implanted in respective bone segments 2a-2d.
In each of the embodiments shown and described in this document, the clamp 3 comprises a lower main body 5 and a locking unit 6 for the bone screws 4.
The locking unit 6 is assembly-mounted on the main body 5.
As better illustrated, for example, in figures 2a, 3a, 4a and 5a, the locking unit 6 comprises two valves 7, 8, respectively lower and upper, designed to engage in order to grip one or more bone screws 4 in respective housings.
As shown in figures 2c, 3c, 4d and 5c, the locking unit 6 comprises two screws 9, only one of which can be fully seen in these figures, designed both to grip the valves 7, 8 together and to fix the mutual position between the valves 7, 8 and the main body 5.
Except for what is shown in figures 2a-2c, wherein the screws are screwed directly into the valve 8 integral with the main body 5, each screw 9 is engaged by screwing with a respective nut 11.
The clamp 3 shown in figures 3a to 3f presents the lower valve 8 provided with a lower concave curve surface 8a.
This lower curved surface 8a is coupled with a convex cylindrical wall 10, integral with the main body 5.

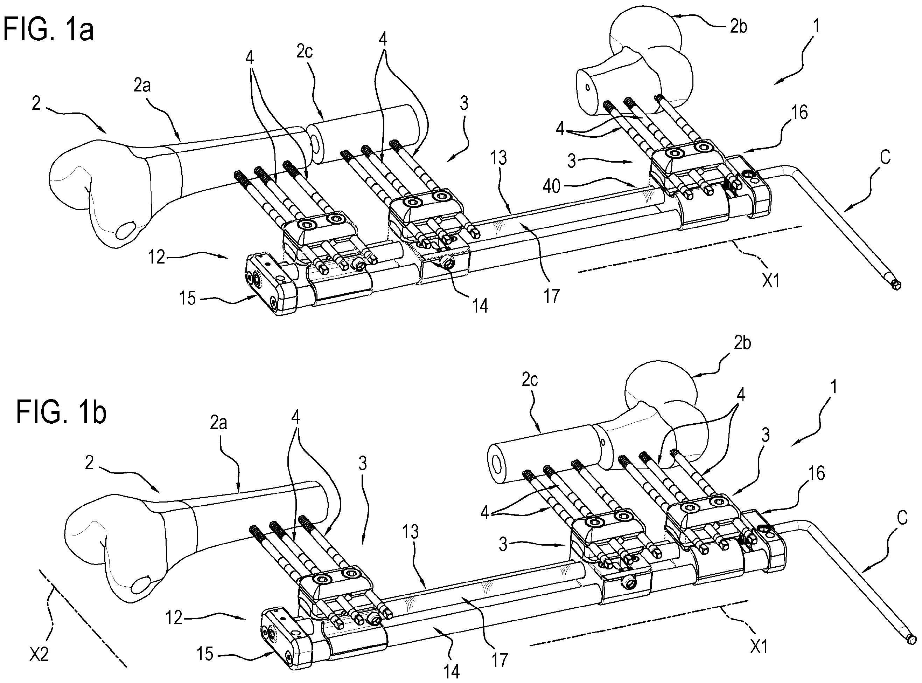

More specifically, with reference to figure 3c, the screw 9 counter engages, with its head 9a, with a lower face of the convex cylindrical wall 10.
Advantageously, with just one fixing mechanism, consisting of the screws 9, it is possible to clamp the valves 7, 8 together and with respect to the main body 5.
The lower curved surface 8a and the cylindrical wall 10 are counter-shaped in order to slide one against the other.
The relative movement of the locking unit 6 with respect to the main body 5, carried out by sliding the lower curved surface 8a on the cylindrical wall 10, defines a rocking movement with respect to a determined axis B, shown in figure 3c.
As shown in figures 1a and 1b, the device 1 comprises a longitudinal guide mechanism 12 positioned externally and aligned with the bone 2 to be treated, the clamps 3 being slidingly engaged on the guide mechanism 12.
The guide mechanism 12 comprises two cylindrical bars 13, 14, parallel to each other and developing longitudinally according to a determined direction X1.
The bars 13, 14 are connected, at their respective opposite ends, to two terminal elements 15, 16.
The guide mechanism 12 also comprises a threaded rod 17, substantially interposed between the cylindrical bars 13, 14 and parallel to them.
The threaded bar 17 is rotatingly engaged at its ends with the terminal elements 15, 16.
At at least one of these terminal elements 15, 16, the threaded rod 17 presents a notch, not shown, to house and engage with an adjustment key C, by means of which a user can rotate the threaded rod 17.
Advantageously, a snap mechanism allows the user to easily calculate the extent of the rotation carried out on the threaded rod 17.

With reference to figures 3a and 3c, the main body 5 of the clamp 3 presents respective first gauged though holes 18, 19, inside which the cylindrical bars 13, 14 slidingly engage, respectively, as shown in figure 1. The main body 5 of the clamp 3 also has a second gauged hole 20 inside which the threaded rod 17 engages.
As shown in detail in figures 2b and 2c, but present in all the variations of the clamp 3, the main body 5 slidingly houses a plug 21 which comprises a threaded portion 21a facing the threaded rod 17 and designed to engage by screwing with it.
The threaded rod 17 together with the threaded portion 21a of the plug 21 defines, for the device 1, respective means 40 for the movement of the clamps 3 along the guide mechanism 12.
The plug 21 is provided with a through hole which houses a screw 22 presenting a threaded area 23 which engages by screwing with the main body 5.
A spring, not shown, is interposed between the main body 5 and the plug 21.
Advantageously, said spring is of the helical type.
The plug 21 and the relative threaded portion 21a are mobile between a first forward configuration, visible in figure 2b, of engagement with the threaded rod 17 and a second rearward configuration, visible in figure 2c, of disengagement with the rod 17.
In the second disengagement position, the rod 17 does not engage by screwing with the threaded portion 21a.
The switch from the first forward position to the second rearward position is carried out by unscrewing the screw 22 so that the plug 21, with its threaded portion 21a, disengages from the threaded rod 17.
Said not shown spring pushes the plug 21 toward its rearward configuration.
The screw 22 defines, for the device 1, means 24 for actuating the threaded portion 21a.

As shown in detail in figure 3c, but present in all the embodiments of the clamp 3, this clamp 3 comprises a sliding block 25 which moves between at least a first configuration for braking the clamp 3, wherein one contact portion 26 interferes with the cylindrical bars 13, 14, and at least a second release position wherein it does not interfere with the cylindrical bars 13, 14.
More specifically, the sliding block 25 is housed in an appropriate cavity in the main body 5 of the clamp 3.
The sliding block 25 presents two cylindrical portions 25a, 25b which together define the contact portion 26.
The cylindrical portions 25a, 25b are designed to at least partially penetrate inside the first gauged holes 18, 19 to come into contact with the cylindrical bars 13, 14 sliding inside them.
A screw 27 screwed into the main body 5 is designed to push its head 27a against the sliding block 25 towards the central body 5, so that the two cylindrical portions 25a, 25b come into contact with the cylindrical bars 13, 14.
This contact between the two cylindrical portions 25a, 25b of the sliding block 25 and the bars 13, 14 determines the onset of friction thus ensuring a stable positioning, or locking, of the relative clamp 3 with respect to the guide mechanism 12.
For the orthopedic device 1, the sliding block 25, together with the screw 27, defines means 33 for locking the clamps 3 with respect to the guide mechanism 12.
With reference to figures 3a to 3f, the clamp 3 shown therein differs from the clamp in figures 2a to 2c basically due to the above-described tendency of the locking unit 6 to rock with respect to the main body 5.
Advantageously, the rocking movement of the locking unit 6 with respect to the main body 5 around the axis B, the line of which can be seen in figure 3d, allows the clamp 3 to adapt to the actual and/or desired position of the bone segment 2a-2d to which its 4 screws are connected.

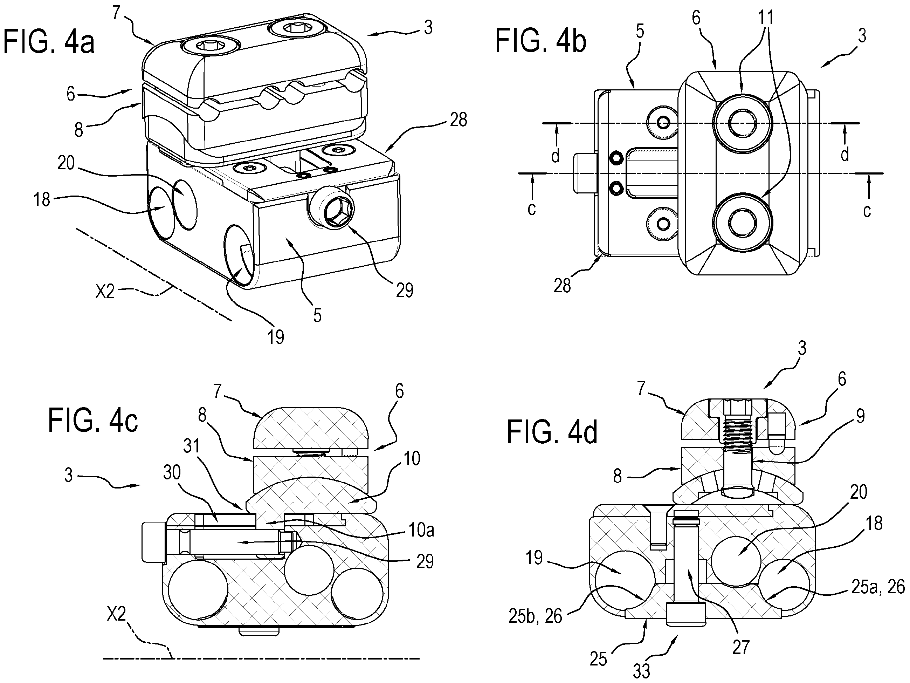

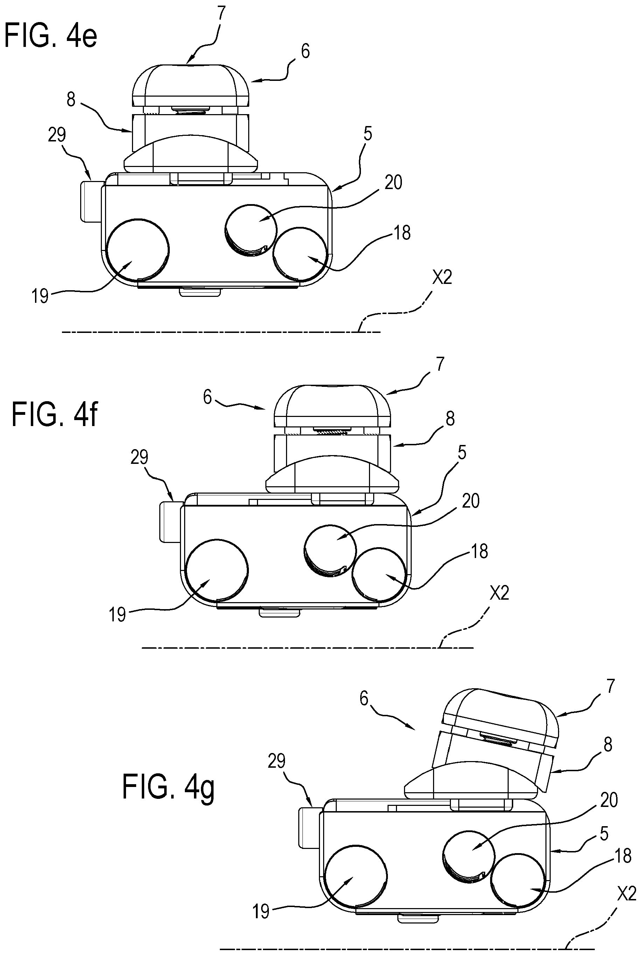

This opportunity contributes to increase the usability of the orthopedic device 1 according to the invention.
With reference to figures 4a to 4g, the clamp 3 shown therein differs from the clamp in figures 2a to 2c basically due to the tendency of the locking unit 6 to move with respect to the main body 5 along a direction X2 substantially at right angles to the aforesaid direction of development of the guide mechanism 12.
With particular reference to figures 4c to 4g, the clamp 3 presents its cylindrical convex wall 10 slidingly engaged on a corresponding dovetail track 28.
A screw 29, visible in figure 4c, is engaged by screwing with a portion 10a of the cylindrical wall 10 which protrudes downwards and is housed in a slot-like cavity 30.
The screw 29 together with the aforesaid downward-protruding portion 10a define a screw-nut coupling which enables the movement of the locking unit 6 according to the direction X2.
Practically speaking, by screwing the downward-protruding portion 10a of the cylindrical wall 10, the screw 29 pulls the locking unit 6 along the aforesaid direction X2.
The slot-like cavity 30 allows the translation movement of the protruding portion 10a.
Advantageously, the translation of the locking unit 6 with respect to the main body 5 along the direction X2 allows the clamp 3 to adapt to the actual and/or desired position of the bone segment 2a-2d to which its 4 screws are connected.
This opportunity contributes to increase the usability of the orthopedic device 1 according to the invention.
Figures 4e to 4g show three different positions of the locking unit 6 along the direction X2 with respect to the main body 5.

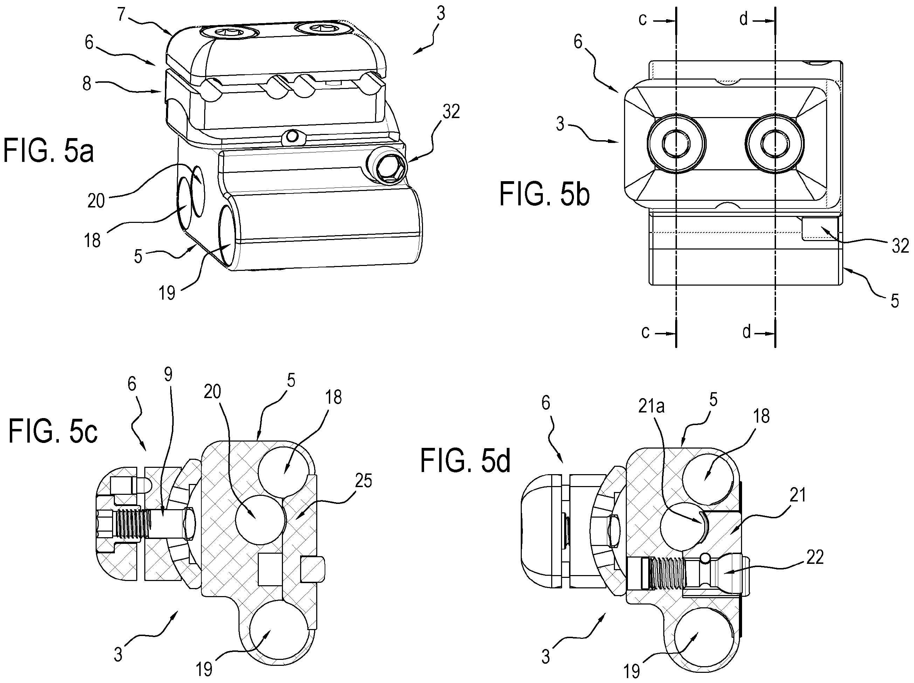

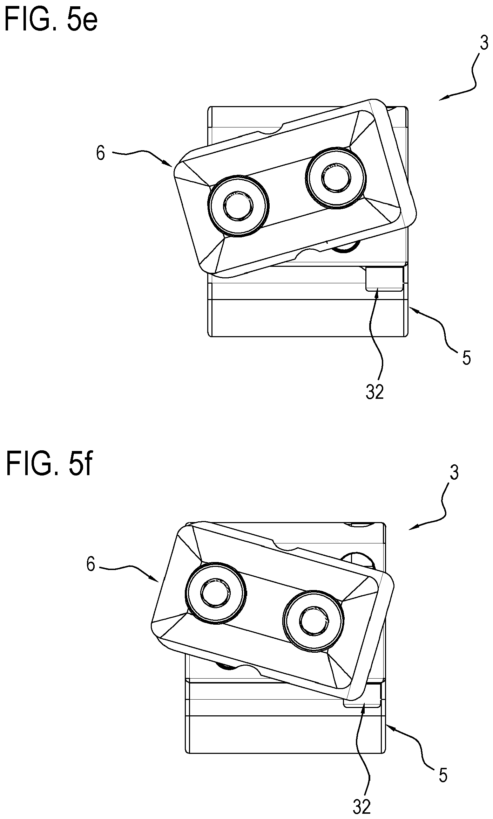

The translation movement described above is carried out by using a normal spanner or key, not shown, designed to engage with the head of the screw 29.
The screw 29 and the protruding portion 10a with which it engages by screwing define means 31 for activating this translation movement.
With reference to figures 5a to 5f, the clamp 3 shown therein differs from the clamp in figures 2a to 2c basically due to the tendency of the locking unit 6 to rotate with respect to the main body 5 along an axis, not shown, substantially perpendicular to the central axis of the threaded rod 17.
With particular reference to figures 5e and 5f, the locking unit 6 rotates integrally with the cylindrical convex wall 10 around a fulcrum pin, not shown.
This rotation is controlled by means of a screw 32, also operated by a known type of spanner or key and not shown.
When used, the orthopedic device 1 according to the invention allows the selective movement of the clamps 3 along the guide mechanism.
In fact, users whose doctors have provided them with a detailed treatment plan can rotate the threaded rod 17 by means of a key C.
Before rotating the threaded rod 17, the user must select the clamp 3 to be moved, disengage the sliding block 25 and engage the threaded portion 21a with the rod 17.
In other words, to place the selected clamp 3 in the condition to be moved by the rod 17 along the cylindrical bars 13, 14, it is necessary on one hand to disengage the clamp 3 from the bars 13, 14 and on the other hand to engage it with the rod 17.
To release the clamp 3, the user acts on the locking means 33 by unscrewing the screw 27 which loosens the contact portion 26 from the bars 13, 14 thereby reducing the friction on them.
To re-engage the clamp 3 with the threaded rod 17, the user tightens the screw 22 in order to move the threaded portion 21a from its second rearward configuration of disengagement to its first configuration of engagement with the threaded rod 17.
In addition, thanks to the multiple possibilities of movement of the locking unit 6 with respect to the main body 5 of the clamp 3, described above and shown in the accompanying drawings, the orthopedic device 1 according to the invention allows effective and functional implant of the device 1 by the surgeon.
Examples of possible movements that can be made on the clamps 3 are shown in the accompanying drawings.
This invention fulfils the preset aims.
In particular, a first advantage connected with the invention consists of the presence of a particularly useful guide mechanism which is easy to use thanks to the operation of all the clamps 3 by means of a single mechanism consisting of the threaded rod 17.
A further advantage of the invention is the easy cleaning of the guide mechanism 12 which, thanks to the presence of the cylindrical bars 13, 14, does not present particular receptacles for the accumulation of dirt or germs.

## Claims

1. External orthopedic device for limb bone treatment, comprising:
- a longitudinal guide mechanism (12) designed to be positioned externally, aligned with the bone (2) to be treated,
- a first clamp (3) holding at least one bone screw (4) to be implanted in a relative bone stump (2a-2d),
- a second clamp (3) holding at least one bone screw (4) to be implanted in a relative bone stump (2a-2d), the clamps (3) being slidingly mobile along the guide mechanism (12),
- means (40) for the movement of the clamps (3) along the guide mechanism (12) to determine a corresponding movement of the bone stumps (2a-2d), wherein the means (40) of movement comprise a threaded rod (17) rotatingly supported by the guide mechanism (12) and a respective threaded portion (21a) in at least one of the clamps (3), the threaded portion (21a) being designed to engage by screwing with the threaded rod (17) to define a screw-nut coupling, **characterised in that** the threaded portion (21a) is mobile at least between a first configuration of engagement with the threaded rod (17) and a second configuration of disengagement from the threaded rod (17), at which point the threaded rod (17) does not engage by screwing with the threaded portion (21a).

2. A device according to claim 1, **characterised in that** it comprises means (24) for transmitting motion to the threaded portion (21a) in order to move it between the first and second configurations.

3. A device according to claim 2, **characterized in that** the means (24) for transmitting motion comprise a screw (22) acting on the threaded portion (21a) by thrust so that it takes up the first engagement configuration.

4. A device according to any of the foregoing claims from 1 to 3, wherein each clamp (3) comprises a main body (5) slidingly engaged with the guide mechanism (12), **characterised in that** the guide mechanism (12) comprises two cylindrical bars (13, 14) positioned parallel to each other and to the threaded rod (17) thereby defining respective sliding tracks for the main body of the clamp (3).

5. A device according to claim 4, **characterised in that** the main body (5) of the clamp (3) presents respective first gauged holes (18, 19) inside which the cylindrical bars (13, 14) slidingly engage.

6. A device according to claim 4 or 5, **characterised in that** the main body (5) of the clamp (3) has a second gauged hole (20) inside which the threaded rod (17) is designed to slide.

7. A device according to any one of the foregoing claims from 4 to 6, **characterised in that** it comprises means (33) for blocking the clamps (3) with respect to the guide mechanism (12), these blocking means (33) acting by friction on at least one of the cylindrical bars (13, 14).

8. A device according to claim 7, **characterised in that** the blocking means (33) comprise a sliding block (25) which moves between at least a first configuration for braking the clamp (3) wherein one portion (26) interferes with at least one of the cylindrical bars (13, 14) and at least a second release position wherein it does not interfere with the cylindrical bars (13, 14).

9. A device according to any of the foregoing claims from 4 to 8, wherein the clamp (3) comprises a unit (6) for locking the at least one bone screw (4) held by the main clamp body (5), **characterised in that** the locking unit (6) can move with respect to the main body (5) in order to adjust the orientation of the bone screw (4) with respect to the longitudinal guide mechanism (12).

10. A device according to claim 9, **characterised in that** the locking unit (6) rocks with respect to the main body (5) according to an axis (B) which is at right angles to the direction in which the bone screw (4) develops.

11. A device according to claim 9 or 10, **characterised in that** the locking unit (6) is designed to move with respect to the main body (5) according to an established direction (X2).

12. A device according to claim 11, **characterised in that** it comprises means (31) for transmitting the motion between the locking unit and the main body.

## Patentansprüche

1. Externe orthopädische Vorrichtung zur Behandlung von Extremitätenknochen, umfassend:
- einen Längsführungsmechanismus (12), der so ausgelegt ist, dass er außen positioniert und mit dem zu behandelnden Knochen (2) ausgerichtet ist,
- eine erste Klammer (3), die mindestens eine Knochenschraube (4) hält, die in einen relativen Knochenstumpf (2a-2d) implantiert werden soll,
- eine zweite Klammer (3), die mindestens eine Knochenschraube (4) hält, die in einen relativen Knochenstumpf (2a-2d) implantiert werden soll, wobei die Klammern (3) entlang des Führungsmechanismus (12) gleitbeweglich sind,
- Mittel (40) zur Bewegung der Klammern (3) entlang des Führungsmechanismus (12) zur Ermittlung einer entsprechenden Bewegung der Knochenstümpfe (2a-2d), wobei die Bewegungsmittel (40) eine Gewindestange (17) umfassen, die durch den Führungsmechanismus (12) und einen jeweiligen Gewindeabschnitt (21a) in mindestens einer der Klammern (3) drehbar gelagert ist, wobei der Gewindeabschnitt (21a) zum Eingreifen durch Verschrauben mit der Gewindestange (17) ausgelegt ist, um eine Schraubenmutterkupplung zu definieren, **dadurch gekennzeichnet, dass** der Gewindeabschnitt (21a) mindestens zwischen einer ersten Konfiguration des Eingriffs mit der Gewindestange (17) und einer zweiten Konfiguration des Lösens von der Gewindestange (17) beweglich ist, an welcher Stelle die Gewindestange (17) durch Verschrauben mit dem Gewindeabschnitt (21a) nicht eingreift.

2. Vorrichtung nach Anspruch 1, **dadurch gekennzeichnet, dass** sie Mittel (24) zur Bewegungsübertragung auf den Gewindeabschnitt (21a) umfasst, um ihn zwischen der ersten und der zweiten Konfiguration zu bewegen.

3. Vorrichtung nach Anspruch 2, **dadurch gekennzeichnet, dass** die Mittel (24) zur Bewegungsübertragung eine Schraube (22) umfassen, die durch Schub auf den Gewindeabschnitt (21a) einwirkt, so dass sie die erste Eingriffskonfiguration einnimmt.

4. Vorrichtung nach einem der vorhergehenden Ansprüche 1 bis 3, wobei jede Klammer (3) einen Hauptkörper (5) umfasst, der gleitend mit dem Führungsmechanismus (12) in Eingriff steht, **dadurch gekennzeichnet, dass** der Führungsmechanismus (12) zwei zylindrische Stangen (13, 14) umfasst, die parallel zueinander und zur Gewindestange (17) positioniert sind, wodurch jeweilige Gleitbahnen für den Hauptkörper der Klammer (3) definiert werden.

5. Vorrichtung nach Anspruch 4, **dadurch gekennzeichnet, dass** der Hauptkörper (5) der Klammer (3) jeweilige erste Messlöcher (18, 19) aufweist, innerhalb derer die zylindrischen Stangen (13, 14) gleitend eingreifen.

6. Vorrichtung nach Anspruch 4 oder 5, **dadurch gekennzeichnet, dass** der Hauptkörper (5) der Klammer (3) ein zweites Messloch (20) aufweist, innerhalb dessen die Gewindestange (17) zu gleiten ausgelegt ist.

7. Vorrichtung nach einem der vorhergehenden Ansprüche 4 bis 6, **dadurch gekennzeichnet, dass** sie Mittel (33) zum Blockieren der Klammern (3) in Bezug auf den Führungsmechanismus (12) umfasst, wobei diese Blockierungsmittel (33) durch Reibung an mindestens einer der zylindrischen Stangen (13, 14) einwirken.

8. Vorrichtung nach Anspruch 7, **dadurch gekennzeichnet, dass** die Blockierungsmittel (33) einen Gleitblock (25) umfassen, der sich zwischen mindestens einer ersten Konfiguration zum Bremsen der Klammer (3), wobei ein Abschnitt (26) mit mindestens einer der zylindrischen Stangen (13, 14) interferiert, und mindestens einer zweiten Freigabeposition, in der sie mit den zylindrischen Stangen (13, 14) nicht interferiert, bewegt.

9. Vorrichtung nach einem der vorhergehenden Ansprüche 4 bis 8, wobei die Klammer (3) eine Einheit (6) zum Verriegeln der mindestens einen Knochenschraube (4) umfasst, die von dem Hauptklammerkörper (5) gehalten wird, **dadurch gekennzeichnet** die Verriegelungseinheit (6) sich in Bezug auf den Hauptkörper (5) bewegen kann, um die Ausrichtung der Knochenschraube (4) in Bezug auf den Längsführungsmechanismus (12) einzustellen.

10. Vorrichtung nach Anspruch 9, **dadurch gekennzeichnet, dass** die Verriegelungseinheit (6) in Bezug auf den Hauptkörper (5) gemäß einer Achse (B) schwingt, die rechtwinklig zu der Richtung ist, in der sich die Knochenschraube (4) erstreckt.

11. Vorrichtung nach Anspruch 9 oder 10, **dadurch gekennzeichnet, dass** die Verriegelungseinheit (6) so ausgelegt ist, dass sie sich in Bezug auf den Hauptkörper (5) gemäß einer festgelegten Richtung (X2) bewegt.

12. Vorrichtung nach Anspruch 11, **dadurch gekennzeichnet, dass** sie Mittel (31) zur Bewegungsübertragung zwischen der Verriegelungseinheit und dem Hauptkörper umfasst.

## Revendications

1. Dispositif orthopédique externe pour traitement d'os de membre, comprenant :
- un mécanisme de guidage longitudinal (12) conçu pour être placé à l'extérieur, aligné avec l'os (2) à traiter,
- une première agrafe (3) maintenant au moins une vis à os (4) à implanter dans un moignon osseux relatif (2a-2d),
- une seconde agrafe (3) maintenant au moins une vis à os (4) à implanter dans un moignon osseux relatif (2a-2d), les agrafes (3) étant mobiles de manière coulissante le long du mécanisme de guidage (12),
- des moyens (40) pour le mouvement des agrafes (3) le long du mécanisme de guidage (12) pour déterminer un mouvement correspondant des moignons osseux (2a-2d), dans lequel les moyens (40) de mouvement comprennent une tige filetée (17) soutenue de manière rotative par le mécanisme de guidage (12) et une portion filetée respective (21a) dans au moins l'une des agrafes (3), la portion filetée (21a) étant conçue pour se mettre en prise par vissage avec la tige filetée (17) pour former un accouplement à écrou de vis, **caractérisé en ce que** la portion filetée (21a) est mobile au moins entre une première configuration d'engagement avec la tige filetée (17) et une seconde configuration de désengagement de la tige filetée (17), en correspondance de laquelle la tige filetée (17) ne se met pas en prise par vissage avec la portion filetée (21a).

2. Dispositif selon la revendication 1, **caractérisé en ce qu'**il comprend des moyens (24) de transmission du mouvement à la portion filetée (21a) de sorte à la déplacer entre la première et la seconde configurations.

3. Dispositif selon la revendication 2, **caractérisé en ce que** les moyens (24) de transmission du mouvement comprennent une vis (22) agissant sur la portion filetée (21a) par poussée de manière qu'elle se mette dans la première configuration d'engagement.

4. Dispositif selon l'une quelconque des revendications précédentes de 1 à 3, dans lequel chaque agrafe (3) comprend un corps principal (5) mis en prise de manière coulissante avec le mécanisme de guidage (12), **caractérisé en ce que** le mécanisme de guidage (12) comprend deux barres cylindriques (13, 14) placées en parallèle l'une par rapport à l'autre et par rapport à la tige filetée (17), formant ainsi des voies de coulissement respectives pour le corps principal de l'agrafe (3).

5. Dispositif selon la revendication 4, **caractérisé en ce que** le corps principal (5) de l'agrafe (3) présente des premiers trous calibrés respectifs (18, 19) dans lesquels les barres cylindriques (13, 14) se mettent en prise de manière coulissante.

6. Dispositif selon la revendication 4 ou 5, **caractérisé en ce que** le corps principal (5) de l'agrafe (3) a un second trou calibré (20) dans lequel la tige filetée (17) est conçue pour coulisser.

7. Dispositif selon l'une quelconque des revendications précédentes de 4 à 6, **caractérisé en ce qu'**il comprend des moyens (33) de blocage des agrafes (3) par rapport au mécanisme de guidage (12), ces moyens de blocage (33) agissant par frottement sur au moins l'une des barres cylindriques (13, 14).

8. Dispositif selon la revendication 7, **caractérisé en ce que** les moyens de blocage (33) comprennent un bloc de coulissement (25) se déplaçant entre au moins une première configuration pour freiner l'agrafe (3), dans laquelle une portion (26) interfère avec au moins l'une des barres cylindriques (13, 14), et au moins une seconde position de libération dans laquelle elle n'interfère pas avec les barres cylindriques (13, 14).

9. Dispositif selon l'une quelconque des revendications précédentes de 4 à 8, dans lequel l'agrafe (3) comprend une unité (6) pour verrouiller ladite au moins une vis à os (4) maintenue par le corps principal de l'agrafe (5), **caractérisé en ce que** l'unité de verrouillage (6) peut se déplacer par rapport au corps principal (5) pour corriger l'orientation de la vis à os (4) par rapport au mécanisme de guidage longitudinal (12).

10. Dispositif selon la revendication 9, **caractérisé en ce que** l'unité de verrouillage (6) balance par rapport au corps principal (5) selon un axe (B) orthogonal à la direction dans laquelle la vis à os (4) se développe.

11. Dispositif selon la revendication 9 ou 10, **caractérisé en ce que** l'unité de verrouillage (6) est conçue pour se déplacer par rapport au corps principal (5) selon une direction établie (X2).

12. Dispositif selon la revendication 11, **caractérisé en ce qu'**il comprend des moyens (31) de transmission du mouvement entre l'unité de verrouillage et le corps principal.
